(19) Europäisches Patentamt
European Patent Office
Office européen des brevets

(11) Veröffentlichungsnummer : **0 149 840**
**B1**

(12) # EUROPÄISCHE PATENTSCHRIFT

(45) Veröffentlichungstag der Patentschrift : **26.04.89**

(51) Int. Cl.⁴ : **C 07 D401/06**, C 07 D223/20, A 61 K 31/55

(21) Anmeldenummer : **84116184.7**

(22) Anmeldetag : **22.12.84**

(54) **Substituierte 5,11-Dihydro-6H-dibenz(b,e)azepin-6-one, Verfahren zu ihrer Herstellung und diese Verbindungen enthaltende Arzneimittel.**

(30) Priorität : **21.01.84 DE 3402060**

(43) Veröffentlichungstag der Anmeldung : **31.07.85 Patentblatt 85/31**

(45) Bekanntmachung des Hinweises auf die Patenterteilung : **26.04.89 Patentblatt 89/17**

(84) Benannte Vertragsstaaten :
**AT BE CH DE FR GB IT LI LU NL SE**

(56) Entgegenhaltungen :
**DE–A– 1 795 176**
**DE–A– 2 918 832**
**DE–A– 3 204 157**
**US–A– 4 308 207**
**CHEMICAL ABSTRACTS, vol. 91, no. 23, 3. Dezember 1979, Columbus, Ohio, USA. EIDEN, FRITZ; DUERR, MANFRED: "Synthesis and properties of 1- and 10-amino-11H-5,6-dihydrodibenz[b,e]azepine-6,11-diones", Seite 630, Spalte 1, Zusammenfassung-Nr. 193 150j**
**CHEMCIAL ABSTRACTS, vol. 94, no. 19 , 11. Mai 1981, Columbus, Ohio, USA. SASAKURA, KAZUYUKI; SUGASAWA, TSUTOMU: "Synthesis of 11-phenyl-5.6-dihydro-11H-dibenz(b,e)azepine derivatives", Seite 641, Spalte 1, Zusammenfassung-Nr. 156 722p**
**CHEMICAL ABSTRACTS, vol. 95, no. 21, 23. November 1981, Columbus, Ohio, USA. LEHUEDE, JACQUES; VIERFOND, JEAN MICHEL; MIOCQUE, MARCEL: "Aminomethylation of homoacridan, iminodibenzyl, and iminostilbene", Seite 634, Spalte 2, Zusammenfassung-Nr. 187 040q**
**Die Akte enthält technische Angaben, die nach dem Eingang der Anmeldung eingereicht wurden und die nicht in dieser Patentschrift enthalten sind.**

(73) Patentinhaber : **Dr. Karl Thomae GmbH**
**Postfach 1755**
**D-7950 Biberach (Riss) (DE)**

(72) Erfinder : **Eberlein, W., Dr., Dipl.-Chem.**
**Obere Au 6**
**D-7950 Biberach 1 (DE)**
Erfinder : **Trummlitz, G., Dr., Dipl.-Chem.**
**Buchenweg 27**
**D-7951 Warthausen (DE)**
Erfinder : **Engel, W., Dr., Dipl.-Chem.**
**Mozartstrasse 13**
**D-7950 Biberach 1 (DE)**
Erfinder : **Schmidt, G., Dr., Dipl.-Chem.**
**Johann-Seb.-Bach-Strasse 27**
**D-7950 Biberach 1 (DE)**
Erfinder : **Hammer, Rudolf, Dr.**
**Via Fabio Filzi 33**
**Milano (IT)**
Erfinder : **Giachetti, Antonio, Prof., Dr.**
**Guerrini 3**
**Milano (IT)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 149 840 B1**

Jouve, 18, rue St-Denis, 75001 Paris, France

**Beschreibung**

Die Erfindung betrifft neue substituierte 5,11-Dihydro-6H-dibenz [b,e] azepin-6-one, Verfahren zu ihrer Herstellung und diese Verbindungen enthaltende Arzneimittel.

In der DT-OS 1 795 176 und DT-OS 3 204 157 werden bestimmte Dibenzodiazepinone mit einer ulcushemmenden und sekretionshemmenden Wirkung beschrieben. Aus der US-PS 3 953 430 sind substituierte Dibenzodiazepine mit antidepressiver und analgetischer Wirkung bekannt. Diese Verbindungen besitzen als Diazepin-Derivate ein anderes Ringsystem.

Es wurde nun gefunden, daß bestimmte 5,11-Dihydro-6H-dibenz [b,e] azepin-6-one gegenüber den Dibenzodiazepinonen bzw. Dibenzodiazepinen der obengenannten Publikationen interessante und pharmakologisch überlegene Wirkungen aufweisen.

Die 5,11-Dihydro-6H-dibenz [b,e] azepin-6-one besitzen die allgemeine Formel

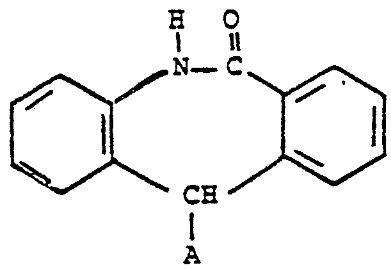

(I)

in der A eine (1-Methyl-4-piperidinyl)acetyl-, (4-Methyl-1-piperazinyl)acetyl- oder [(1-Methyl-4-piperidinyl)amino]-carbonylgruppe bedeutet.

Die Verbindungen der allgemeinen Formel I können nach Umsetzung mit anorganischen oder organischen Säuren auch in Form ihrer physiologisch verträglichen Salze vorliegen. Als Säuren haben sich hierzu beispielsweise Salzsäure, Bromwasserstoffsäure, Schwefelsäure, Phosphorsäure, Weinsäure, Fumarsäure, Zitronensäure, Maleinsäure, Bernsteinsäure, Gluconsäure, Äpfelsäure, p-Toluolsulfonsäure, Methansulfonsäure oder Amidosulfonsäure als geeignet erwiesen.

Im einzelnen betrifft die Erfindung die folgenden Verbindungen :

5,11-Dihydro-11-[(1-methyl-4-piperidinyl)acetyl]-6H-dibenz [b,e] azepin-6-on,
5,11-Dihydro-11-[(4-methyl-1-piperazinyl)acetyl]-6H-dibenz [b,e] azepin-6-on und
5,11-Dihydro-11-[[(1-methyl-4-piperidinyl)amino]carbonyl]-6H-dibenz [b,e] azepin-6-on

sowie deren physiologisch unbedenkliche Salze.

Gegenstand der Erfindung sind auch Arzneimittel, die ein oder mehrere Dibenzazepinone der allgemeinen Formel I enthalten.

Die Verbindungen der allgemeinen Formel I lassen sich hierzu in an sich bekannter Weise in die üblichen pharmazeutischen Zubereitungsformen, z. B. in Lösungen, Suppositorien, Tabletten, Dragées, Kapseln oder Teezubereitungen einarbeiten. Die Tagesdosis liegt im allgemeinen zwischen 0,01 und 5, vorzugsweise 0,02 und 2,5, insbesondere 0,05 und 1,0 mg/kg Körpergewicht, die gegebenenfalls in Form mehrerer, vorzugsweise 1 bis 3 Einzelgaben, zur Erzielung der gewünschten Ergebnisse verabreicht wird.

Die substituierten Dibenzazepinone der allgemeinen Formel I und ihre Säureadditionssalze besitzen wertvolle Eigenschaften, die sie gewerblich verwertbar machen, insbesondere sind sie durch eine ausgezeichnete Magen- und Darmschutzwirkung bei Warmblütern gekennzeichnet, sie hemmen z. B. die Bildung von Magengeschwüren. Ferner weisen sie, bedingt durch eine geringe Toxizität und das Fehlen wesentlicher Nebenwirkungen eine günstige therapeutische Breite auf.

Die ausgezeichnete Wirksamkeit der substituierten Dibenz-azepinone der allgemeinen Formel I und/oder ihrer pharmakologisch verträglichen Säureadditionssalze ermöglicht ihren Einsatz in der Human- und auch in der Veterinärmedizin, wobei sie zur Behandlung und Prophylaxe von Erkrankungen des Magens oder Darms verwendet werden. Beispielsweise können mit ihnen akuter und chronischer Ulcus ventriculi und duodeni, Gastritis oder hyperacider Reizmagen bei Mensch und Tier behandelt werden.

Sollen die erfindungsgemäßen substituierten Dibenzazepinone der allgemeinen Formel I und/oder ihre pharmakologisch verträglichen Säureadditionssalze zur Behandlung der angegebenen Krankheiten eingesetzt werden, so können die pharmazeutischen Zubereitungen auch einen oder mehrere pharmakologisch aktive Bestandteile anderer Arzneimittelgruppen, wie Antacida, beispielsweise Aluminiumhydroxid, Magnesiumaluminat ; Sekretionshemmer, wie $H_2$-Blocker, z. B. Cimetidin, Ranitidin ; Magen- und Darmtherapeutika, z. B. Metoclopramid, Bromoprid, Tiaprid ; Tranquilizer, wie Benzodiazepine, beispielsweise Diazepam, Oxazepam ; Spasmolytika, z. B. Bietamiverin, Camylofin ; Anticholinergica, z. B. Oxyphencyclimin, Phencarbamid ; Glucocorticoide, wie Prednisolon, Fluocortolon, Betamethason ; nichtsteroidale Antiphlogistika, wie Arylessigsäuren und -propionsäuren, Heteroarylessigsäuren und

-propionsäuren, Benzothiazincarboxamiddioxide, Pyrazolidindione, Chinazolinone, z. B. Ibuprofen, Naproxen, Diclofenac, Fenbufen, Flurbiprofen, Indometacin, Lonazolac, Sudoxicam, Piroxicam, Phenylbutazon, Bumadizon-Calcium, Proquazon ; Lokalanaesthetika, beispielsweise Tetracain, Procain ; gegebenenfalls auch Fermente, Vitamine, Aminosäuren etc. enthalten.

Ein weiterer Gegenstand der Erfindung sind Verfahren zur Herstellung der Dibenzazepinone der allgemeinen Formel I.

Die Verbindungen der allgemeinen Formel I, in der A die (1-Methyl-4-piperidinyl)acetyl- oder (4-Methyl-1-piperazinyl) acetylgruppe bedeutet, erhält man dadurch, daß man zuerst das 5,11-Dihydro-6H-dibenz [b,e] azepin-6-on der Formel II

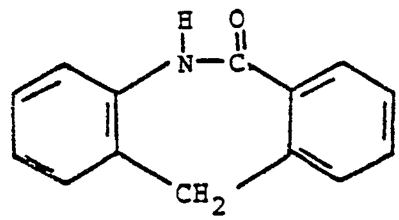

(II)

in sein Di-Lithiumsalz überführt und letzteres anschließend mit einem Ester der allgemeinen Formel III

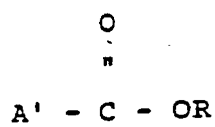

(III)

in der

A′ die (1-Methyl-4-piperidinyl)methyl- oder (4-Methyl-1-piperazinyl)-methylgruppe bedeutet, und

R einen Alkylrest mit 1 bis 10 Kohlenstoffatomen, vorzugsweise 1 bis 6 Kohlenstoffatomen, oder einen Aralkylrest mit 7 bis 13 Kohlenstoffatomen, vorzugsweise einen Phenylalkylrest mit 7 bis 9 Kohlenstoffatomen, darstellt, umsetzt.

Die Überführung des 5,11-Dihydro-6H-dibenz[b,e]azepin-6-ons der Formel II in das Di-Lithiumsalz gelingt mit Lithiumalkylen, insbesondere aber mit n-Butyllithium, n-Butyllithium in Gegenwart von Tetramethylethylendiamin, tertiär Butyllithium, Lithiumdiisopropylamid oder Lithiumdicyclohexylamid oder mit Lithiumarylen, z. B. mit Lithiumphenyl. Die Überführung in das Di-Lithiumsalz und die weitere Umsetzung zu den Verbindungen der allgemeinen Formel I erfolgt in einem inerten organischen Lösungsmittel bei Temperaturen zwischen — 60 °C und Raumtemperatur, vorzugsweise aber zwischen — 10 °C und Raumtemperatur. Als organische Lösungsmittel dienen solche, die für Umsetzungen mit Lithium-alkylen oder Lithiumarylen gebräuchlich sind ; besonders vorteilhaft ist die Verwendung von Tetrahydrofuran oder Ethern, wie Diethylether, von aliphatischen Kohlenwasserstoffen, wie Hexan oder von Gemischen hiervon, gegebenenfalls auch in Gegenwart von Hexamethylphosphorsäuretriamid als Cosolvens. Kurze Zeit nach der Beendigung der Zugabe des Lithiumalkyls oder -aryls gibt man die stöchiometrische Menge oder einen leichten Überschuß des Esters der allgemeinen Formel III hinzu und läßt das Reaktionsgemisch zur Vervollständigung der Reaktion langsam, z. B. innerhalb von 2 Stunden, auf Raumtemperatur kommen. Man isoliert das gebildete Produkt der allgemeinen Formel I nach üblichen Methoden aus dem Reaktionsgemisch und erhält die gesuchte Verbindung in Form ihrer Base, die anschließend gewünschtenfalls in ihre Salze übergeführt werden kann.

Die Verbindung der allgemeinen Formel I, in der A die [(1-Methyl-4-piperidinyl)amino] carbonylgruppe bedeutet, erhält man durch Umsetzung reaktiver Derivate der Carbonsäure der Formel IV

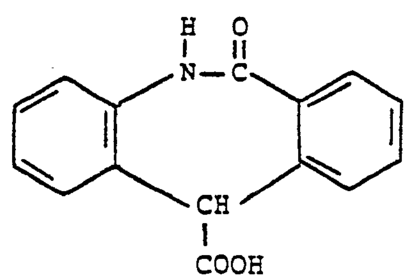

(IV)

mit 1-Methyl-4-amino-piperidin.

Als reaktive Derivate der Carbonsäure der Formel IV kommen bevorzugt die Säurehalogenide,

gemischte Anhydride, z. B. mit der Chlorameisensäure, die Umsetzungsprodukte mit N,N-Carbonyldiimidazol oder mit N,N'-Dicyclohexylcarbodiimid, aber auch ihre Ester, vorzugsweise mit aliphatischen Alkoholen von 1 bis 8 Kohlenstoffatomen oder araliphatischen Alkoholen mit 7 bis 13 Kohlenstoffatomen in Frage.

Die Reaktion der Säurehalogenide und -anhydride oder der Carbonsäure der Formel IV mit dem 1-Methyl-4-amino-piperidin erfolgt in inerten organischen Lösungsmitteln oder in einem Überschuß dieses Amins und zwischen Zimmertemperatur und dem Siedepunkt des Reaktionsgemisches, vorzugsweise bei Temperaturen zwischen 40 und 70 °C. Als Lösungsmittel werden Äther, wie Dioxan, Tetrahydrofuran, aromatische Kohlenwasserstoffe, wie Benzol, Chlorbenzol, Toluol, oder polare Lösungsmittel, wie Dimethylformamid, Hexamethylphosphorsäuretriamid, verwendet.

Arbeitet man mit einem Chlorameisensäurealkylester, so wird die Carbonsäure in einem Lösungsmittel, z. B. einem chlorierten Kohlenwasserstoff, vorzugsweise jedoch in einem Ester einer aliphatischen Carbonsäure in Gegenwart einer Base, z. B. eines Trialkylamins, suspendiert und unter Eiskühlung mit dem Chlorameisensäurealkylester versetzt. Das 1-Methyl-4-amino-piperidin wird mit dem als Zwischenprodukt entstehenden Anhydrid bei Temperaturen zwischen 0 °C und 30 °C zur Reaktion gebracht. Die Isolierung des Endproduktes erfolgt nach an sich bekannten Methoden.

Die Umsetzung mit N,N'-Carbonyldiimidazol oder N,N'-Dicyclohexylcarbodiimid erfolgt in inerten Lösungs- oder Suspensionsmitteln, bevorzugt in Tetrahydrofuran oder Dioxan, und bei Temperaturen zwischen 0 °C und 100 °C, vorzugsweise aber bei 30 bis 60 °C ; das 1-Methyl-4-amino-piperidin wird dann anschließend bei diesen Temperaturen mit den sich bildenden Zwischenverbindungen, ohne vorherige Isolierung derselben, umgesetzt.

Die Reaktion der Ester der Carbonsäure der Formel IV mit dem 1-Methyl-4-amino-piperidin erfolgt ebenfalls in geeigneten, indifferenten organischen Lösungsmitteln, beispielsweise in aromatischen Kohlenwasserstoffen, wie Benzol, Toluol, Xylol, Chlorbenzol, Tetrahydronaphthalin, aber auch in Dimethylformamid, Dimethylacetamid, Dimethylsulfoxyd oder in Hexamethylphosphorsäuretriamid, in Äthern, wie Dioxan, Tetrahydrofuran oder direkt im überschüssigen 1-Methyl-4-amino-piperidin. Die Umsetzung erfolgt bei Temperaturen zwischen 20 und 180 °C, vorzugsweise aber bei der Siedetemperatur des freiwerdenden Alkohols, der vorteilhafterweise gleichzeitig durch azeotrope Destillation entfernt wird.

Die erfindungsgemäßen Dibenzazepinone der allgemeinen Formel I enthalten ein asymmetrisches Kohlenstoffatom. Diese Verbindungen können deshalb in enantiomeren Formen auftreten. Die Erfindung umfaßt die einzelnen Enantiomeren ebenso wie ihre Gemische.

Die Spaltung eventueller Razemate der Verbindungen der allgemeinen Formel I kann nach bekannten Verfahren durchgeführt werden, beispielsweise unter Verwendung einer optisch aktiven Säure, wie (+)- oder (—)-Weinsäure, oder eines Derivates davon, wie (+)- oder (—)-Diacetylweinsäure, (+)- oder (—)-Monomethyltartrat oder (+)-Camphersulfonsäure.

Nach einem üblichen Verfahren zur Isomerentrennung wird das Razemat einer Verbindung der allgemeinen Formel I mit einer der vorstehend beschriebenen optisch aktiven Säuren in äquimolarer Menge in einem Lösungsmittel umgesetzt und die erhaltenen kristallinen optisch aktiven Salze werden unter Ausnutzung ihrer verschiedenen Löslichkeit getrennt. Diese Umsetzung kann in jeder Art von Lösungsmittel durchgeführt werden, solange dieses einen ausreichenden Unterschied in der Löslichkeit der Salze aufweist. Vorzugsweise werden Methanol, Ethanol oder deren Gemische, beispielsweise im Volumenverhältnis 50 : 50 verwendet. Sodann wird jedes der optisch aktiven Salze in Wasser gelöst, neutralisiert und dadurch die entsprechende freie Verbindung in der (+)- oder (—)-Form erhalten. Jeweils nur ein Enantiomeres wird auch dadurch erhalten, daß man das oben angeführte Verfahren b) mit nur einem Enantiomeren der Formel IV durchführt.

Das als Ausgangsverbindung verwendete 5,11-Dihydro-6H-dibenz[b,e]azepin-6-on der Formel II erhält man durch Erhitzen von 2-Benzyl-phenylisocyanat in Gegenwart von wasserfreiem Aluminiumchlorid (vgl. Helv. Chim. Act 48, 336 (1965)). Es läßt sich aber auch leicht durch katalytische Hydrierung aus dem bekannten Morphanthridon herstellen.

Die Carbonsäure der Formel IV erhält man durch Oxidation von 5,11-Dihydro-11-aldehydo-6H-dibenz[b,e]azepin-6-on mit Natrium-dichromat in Eisessig/Schwefelsäure (vgl. K. Ackermann et al., Can. J. Chem. 47, 4327 (1969)). Die Aldehydo-Verbindung wird ihrerseits aus dem bekannten 5,11-Dihydro-6H-dibenz[b,e]azepin-6,11-dion (Morphanthridon) hergestellt. Alternativ läßt sich die Carbonsäure der Formel IV auch nach literaturüblichen Verfahren durch Umsetzung des Dilithiumsalzes der Verbindung der Formel II mit Kohlendioxid erhalten.

Wie bereits erwähnt, weisen die neuen Verbindungen der allgemeinen Formel I wertvolle pharmakologische Eigenschaften auf ; insbesondere besitzen sie antiulcerogene und magensäuresekretionshemmende Wirkungen, sowie günstige Effekte auf verschiedene andere Erkrankungen des Magen-Darm-Traktes, unter denen das Colon irritabile besonders hervorgehoben sei.

Eine günstige Relation zwischen antiulcerogenen und antisekretorischen Wirkungen einerseits und den bei Therapeutika mit anticholinerger Wirkkomponente üblicherweise auftretenden unerwünschten Wirkungen auf die Pupillenweite und Tränen- und Speichelsekretion andererseits ist für die therapeutische Verwendung der Substanzen von besonderer Wichtigkeit. Die folgenden Versuche zeigen, daß die erfindungsgemäßen Verbindungen diesbezüglich überraschend günstige Relationen aufweisen.

Untersuchung auf Selektivität der antimuscarinischen Wirkung

Ziel

Oxotremorin, ein spezifischer Agonist an muscarinischen Rezeptoren, erzeugt bei Ratten Läsionen der Magenschleimhaut und steigert die Speichelsekretion. Dieses Versuchsmodell wurde gewählt, um eine selektive Wirkung einer antimuscarinischen Substanz auf den Magen erkennen zu können.

Methodik

Verwendet wurden 10 weibliche Albino-Ratten (Stamm Crl : COBS-CD (SD) BR) pro Behandlungsgruppe mit einem Körpergewicht von 120 bis 150 g, die bei freiem Zugang zu Trinkwasser 24 Stunden vor Versuchsbeginn ohne Nahrung waren.

Um in Vorversuchen die muscarinische Wirkung von Oxotremorin auf jedes der untersuchten Symptome zu ermitteln, wurde mit jeweils mindestens drei Dosen für jedes Symptom eine Dosis-Wirkungskurve erstellt.

Bei der Prüfung antimuscarinischer Substanzen wurde die Oxotremorin-Dosis gewählt, die in den Vorversuchen das zu beeinflussende Symptom bei 90 bis 100 % der Tiere ausgelöst hatte.

| | |
|---|---|
| Magenschleimhaut-Läsionen | 0,62 mg/kg i.v. |
| Speichselsekretion | 0,083 mg/kg i.v. |

Jede antimuscarinische Substanz wurde in gleichmäßig abgestuften Dosierungen 15 Minuten vor der Oxotremorin-Gabe intravenös verabreicht. Kontrollgruppen erhielten an Stelle der Prüfsubstanz das Lösungs- und Suspensionsmittel in entsprechender Menge.

Unmittelbar nach Oxotremorin-Gabe wurden die Tiere in einem Glaskäfig für 15 Minuten beobachtet.

Die Prüfung auf Beeinflussung der Oxotremorin-induzierten Speichelsekretion wurde blind durchgeführt, d.h. der Untersucher wußte nicht, wie die Tiere vorbehandelt waren.

Die Ergebnisse wurden als prozentuale Hemmung des Oxotremorin-Effektes (Prozentsatz der Tiere ohne das entsprechende Symptom) ausgedrückt. $ED_{50}$-Werte wurden nach der Methode von Litchfield und Wilcoxon (J. Pharmacol. Exp. Ther. 96, 99, 1949) ermittelt.

Die Effekte auf Schleimhaut-Läsionen des Magens wurden folgendermaßen bewertet :

Die Magenschleimhaut-Läsionen wurden erzeugt durch intravenöse Injektion von 0,62 mg/kg Oxotremorin 30 Minuten nach oraler Gabe von 1 mg/kg Neostigmin (Cholinesterase-Hemmer). 60 Minuten nach Neostigmin-Gabe wurden die Tiere getötet, die Mägen entnommen, geöffnet und auf Vorhandensein von Schleimhaut-Läsionen geprüft. Die schützende Wirkung der Prüfsubstanzen wurde als prozentuale Hemmung (Prozentsatz der Tiere ohne Läsionen) ausgedrückt. Die $ED_{70}$-Werte wurden nach der Methode von Litchfield und Wilcoxon (s.o.) ermittelt.

Mydriasis

Der Effekt von Testsubstanzen auf die Pupillenweite von Ratten wurde folgendermaßen untersucht :

Die Substanzen wurden an Gruppen von jeweils 10 Tieren in wenigstens 3 gleichmäßig abgestuften Dosierungen intravenös appliziert. Die Pupillenweite wurde anschließend 10 Minuten lang auf evtl. Veränderungen (Auftreten von Mydriasis oder Miosis) beobachtet, und zwar wiederum blind, d.h. dem jeweiligen Untersucher war die Vorbehandlung der Tiere unbekannt. Es wurde der Prozentsatz der Versuchstiere ermittelt, bei denen eine Mydriasis auftrat. $ED_{50}$-Werte wurden wiederum nach Litchfield und Wilcoxon (s.o.) bestimmt.

Bindungsstudien an muscarinischen Rezeptoren

Bestimmung des $IC_{50}$-Wertes

Als Organspender dienten männliche Sprague-Dawley-Ratten mit 180-220 g Körpergewicht. Nach Entnahme von Magen und Großhirnrinde wurden alle weiteren Schritte im eiskalten Hepes-HCl-Puffer (pH 7,4 : 100 m molar NaCl, 10 m molar $MgCl_2$) vorgenommen. Die glatte Muskulatur des Magenfundus wurde von der Magenschleimhaut freipräpariert und vorhomogenisiert. Alle Organe wurden abschließend in einem Potter homogenisiert.

Für den Bindungstest wurden die Organhomogenate in folgender Weise verdünnt :

| | |
|---|---|
| Glatter Muskel Magenfundus | 1 : 100 |
| Großhirnrinde | 1 : 3 000 |

Die Inkubation der Organhomogenate erfolgte bei einer bestimmten Konzentration des Radioliganden und einer Konzentrationsreihe der nichtradioaktiven Testsubstanzen im Eppendorf-Zentrifugenröhrchen bei 30 °C. Die Inkubationsdauer betrug 45 Minuten. Als Radioligand wurde 0,3 n molar $^3$H-N-Methylscopolamin ($^3$H-NMS) verwendet. Nach Beendigung der Inkubation durch Zentrifugation bei 14 000 g wurde die Radioaktivität im Pellet bestimmt. Sie repräsentiert die Summe von spezifischer und unspezifischer Bindung von $^3$H-NMS. Der Anteil der unspezifischen Bindung wurde definiert als jene Radioaktivität, die in Anwesenheit von 1 μ molar Chinuclidinylbenzilat gebunden wurde. Es wurden immer Vierfachbestimmungen vorgenommen. Die $IC_{50}$-Werte der nichtmarkierten Testsubstanzen wurden graphisch bestimmt. Sie repräsentieren jene Konzentration der Testsubstanz, bei welcher die spezifische Bindung von $^3$H-NMS an die muscarinischen Rezeptoren in den verschiedenen Organen um 50 % gehemmt wurde. Nach den vorstehenden Angaben wurden beispielsweise die folgenden Verbindungen untersucht :

A = 5,11-Dihydro-11-[[(1-methyl-4-piperidinyl)amino]carbonyl-6H-dibenz[b,e]azepin-6-on
B = 5,11-Dihydro-11-[(1-methyl-4-piperidinyl)acetyl]-6H-dibenz[b,e]azepin-6-on
C = 5,11-Dihydro-11-[(4-methyl-1-piperazinyl)acetyl]-6H-dibenz[b,e]azepin-6-on

Ergebnisse

| Substanz | Rezeptor-Bindungs-Tests $IC_{50}$ [n Mol $l^{-1}$] Cortex | Glatter Muskel Magenfundus | Oxotremorin-Tests [μg/kg] i.v. Antiulcerogene Wirkung $ED_{70}$ | Salivationshemmmung $ED_{50}$ | Mydriasis $ED_{50}$ [μg/kg] i.v. |
|---|---|---|---|---|---|
| A | 40 | 700 | 22 | 190 | 75 |
| B | 25 | 150 | 13 | 186 | 179 |
| C | 30 | 400 | 80 | 335 | 161 |

Die Angaben in der vorstehenden Tabelle beweisen, daß die genannten Verbindungen generell eine hohe Affinität zu muscarinischen Rezeptoren besitzen. Darüberhinaus kann man den Daten entnehmen, daß die neuen Verbindungen der allgemeinen Formel I zwischen muscarinischen Rezeptoren verschiedener Gewebe unterscheiden. Dies folgt aus den beträchtlich niedrigeren $IC_{50}$-Werten bei Untersuchung an Präparaten der Großhirnrinde gegenüber solchen aus der glatten Muskulatur des Magens.

Aus den pharmakologischen Daten der vorstehenden Tabelle ergibt sich — in völliger Übereinstimmung mit den Rezeptor-Bindungs-Studien-, daß die Entstehung Oxotremorin-induzierter Magenschleimhaut-Läsionen durch die genannten Verbindungen bereits bei Dosierungen gehemmt wird, bei denen noch keine Einschränkung der Speichelsekretion und keine Mydriasis beobachtet wird.

Die Verbindungen der allgemeinen Formel I sind weitgehend untoxisch noch bei Dosierungen von über 1 000 mg/kg Maus.

Die folgenden Beispiele dienen zur näheren Erläuterung der Erfindung. « F » bedeutet « Schmelzpunkt », « Z. » bedeutet « Zersetzung ».

Beispiel 1

5,11-Dihydro-11-[(1-methyl-4-piperidinyl)acetyl]-6H-dibenz-[b,e]azepin-6-on

Zu einer Lösung von 5 g (0,024 Mol) 5,11-Dihydro-6H-dibenz-[b,e]azepin-6-on in 70 ml absolutem Tetrahydrofuran werden unter Rühren bei Raumtemperatur 25,6 g (0,06 Mol) n-Butyl-lithium (15 %ig in Hexan) zugetropft. Man rührt 30 Minuten bei 40 °C nach und tropft anschließend bei 5 °C 11,1 g (0,06

Mol) 1-Methyl-4-piperidinessigsäureethylester in die Lösung ein. Man läßt 30 Minuten nachrühren und zieht dann das Lösungsmittel im Vakuum ab. Der verbleibende Rückstand wird in wenig Wasser gelöst, mit verdünnter Salzsäure angesäuert und nochmals mit Ether extrahiert. Die Etherauszüge werden verworfen. Die wässrige Phase wird durch Zugabe von Kaliumkarbonat alkalisch gestellt und mit Chloroform erschöpfend extrahiert. Die vereinigten Chloroformauszüge werden mit Magnesiumsulfat getrocknet und im Vakuum zur Trockne eingeengt. Die Reinigung des Rohproduktes erfolgt durch Chromatographie an Kieselgel unter Verwendung eines Gemisches von Chloroform/Essigester/Methanol als Elutionsmittel. Man erhält aus dem Eluat 950 mg (11 % der Theorie) der gewünschten Verbindung, die nach dem Umkristallisieren aus Essigester bei 218-220 °C schmilzt.

## Beispiel 2

5,11-Dihydro-11-[(4-methyl-1-piperazinyl)-acetyl]-6H-dibenz-[b,e]azepin-6-on

Hergestellt analog Beispiel 1 aus 5,11-Dihydro-6H-dibenz-[b,e]azepin-6-on und 4-Methyl-1-piperazinessigsäureethylester in einer Ausbeute von 21 %.

F. 184-186 °C.

## Beispiel 3

5,11-Dihydro-11-[[(1-methyl-4-piperidinyl)amino]carbonyl]-6H-dibenz[b,e]azepin-6-on

2,5 g (0,01 Mol) 5,11-Dihydro-6H-dibenz[b,e]azepin-6-on-11-carbonsäure werden in einer Mischung von 50 ml Chloroform und 15 ml Thionylchlorid bis zur vollständigen Lösung unter Rückfluß erhitzt. Anschließend wird das Lösungsmittel im Vakuum abgezogen und der verbleibende Rückstand in 50 ml Dioxan aufgenommen. Zu dieser Lösung tropft man langsam eine Mischung von 2,2 g (0,02 Mol) 4-Amino-1-methyl-piperidin in 50 ml Dioxan ein und rührt noch 60 Minuten bei 50 °C nach. Man engt im Vakuum ein, versetzt den Rückstand mit wenig Wasser, sättigt die Lösung mit Kaliumkarbonat und extrahiert erschöpfend mit Essigsäureethylester. Die vereinigten Extrakte werden über Aktivkohle filtriert und anschließend im Vakuum zur Trockene eingedampft. Die Reinigung des Rohproduktes erfolgt durch Chromatographie an Kieselgel unter Verwendung von Methanol als Elutionsmittel. Man erhält farblose Kristalle vom F. 230-231 °C in einer Ausbeute von 1,3 g (36 % der Theorie).

F. (Hydrochlorid) : 306-307 °C (Zersetzung).

## Beispiel 4

5,11-Dihydro-11-[[(1-methyl-4-piperidinyl)amino]carbonyl]-6H-dibenz[b,e]azepin-6-on

In eine Suspension von 2,5 g (0,01 Mol) 5,11-Dihydro-6-oxo-6H-dibenz[b,e]azepin-11-carbonsäure und 2,0 g (0,02 Mol) Triäthylamin in 150 ml Essigester werden unter Eiskühlung langsam 1,8 g (0,01 Mol) Chlorameisensäureethylester eingetropft. Man rührt 1 Stunde bei Raumtemperatur nach und gibt dann tropfenweise eine Lösung von 1,25 g (0,01 Mol) 4-Amino-1-methyl-piperidin in 20 ml Essigsäureethylester hinzu. Nach dem Stehen über Nacht schüttelt man die Reaktionslösung mehrmals mit verdünnter Salzsäure aus, trennt die wässrigsauren Auszüge ab und neutralisiert, beispielsweise durch Zugabe von festem Natriumbikarbonat. Man extrahiert die wässrige Lösung erschöpfend mit Essigsäureethylester, trocknet über Magnesiumsulfat und engt im Vakuum zur Trockne ein. Durch Digerieren des Rückstandes in wenig Ether erhält man Kristalle vom F. 230-231 °C in einer Ausbeute von 1,8 g (51 % der Theorie). Die Substanz ist nach Mischschmelzpunkt, Dünnschichtchromatogramm und IR-Spektrum identisch mit dem nach Beispiel 3 hergestellten Präparat.

## Beispiel 5

5,11-Dihydro-11-[[(1-methyl-4-piperidinyl)amino]carbonyl]-6H-dibenz[b,e]azepin-6-on

Zu einer Suspension von 5 g (0,02 Mol) 5,11-Dihydro-6-oxo-6H-dibenz[b,e]azepin-11-carbonsäure in 100 ml Tetrahydrofuran werden 3,5 g (0,022 Mol) N,N'-Carbonyldiimidazol gegeben und das Gemisch 30 Minuten auf 40 °C erwärmt. Anschließend setzt man 2,5 g (0,022 Mol) 4-Amino-1-methylpiperidin zu und erwärmt weitere zwei Stunden auf 40 °C. Nach dem Erkalten wird das Lösungsmittel im Vakuum abgezogen und der Rückstand durch Säulenchromatographie an Kieselgel (Ethylenchlorid/-Methanol = 9/1) gereinigt. Aus dem Eluat erhält man 4,8 g (68 %) der gewünschten Verbindung vom F. 230-231 °C. Die Substanz ist nach Mischschmelzpunkt, Dünnschichtchromatogramm und IR-Spektrum identisch mit dem nach Beispiel 3 hergestellten Präparat.

Beispiel 6

5,11-Dihydro-11-[[(1-methyl-4-piperidinyl)amino]carbonyl]-6H-dibenz[b,e]azepin-6-on

Eine Suspension von 5 g (0,02 Mol) 5,11-Dihydro-6-oxo-6H-dibenz[b,e]azepin-11-carbonsäure und 4,5 g (0,022 Mol) N,N'-Dicyclohexylcarbodiimid in 180 ml Tetrahydrofuran erwärmt man während 60 Minuten auf 40 °C. Anschließend tropft man in die Reaktionslösung 2,5 g (0,022 Mol) 4-Amino-1-methyl-piperidin ein und erwärmt weitere zwei Stunden auf 40-50 °C. Nach dem Erkalten wird die Lösung im Vakuum eingeengt und der verbleibende Rückstand durch Chromatographie an Kieselgel (Methylenchlorid/Methanol = 9/1) gereinigt. Aus dem Eluat erhält man Kristalle vom F. 230-231 °C in einer Ausbeute von 3,7 g (53 % der Theorie). Die Substanz ist nach Mischschmelzpunkt, Dünnschichtchromatogramm und IR-Spektrum identisch mit dem nach Beispiel 3 hergestellten Präparat.

Beispiel 7

5,11-Dihydro-11-[(1-methyl-4-piperidinyl)acetyl]-6H-dibenz-[b,e]azepin-6-on

Analog Beispiel 1 wird eine Lösung von 3,0 g (0,03 Mol) Diisopropylamin in 50 ml Tetrahydrofuran und 21,3 ml (0,032 Mol, 1,6 molar in Hexan) n-Butyllithium bei — 10 °C mit 2,0 g (0,01 Mol) 5,11-Dihydro-6H-dibenz[b,e]azepin-6-on und 5,5 g (0,032 Mol) 1-Methyl-4-piperidinessigsäuremethylester zur Reaktion gebracht.

Nach Reinigung des Rohproduktes durch Chromatographie an Kieselgel erhält man eine Verbindung, die nach Mischschmelzpunkt, Dünnschichtchromatogramm und IR-Spektrum identisch ist mit dem nach Beispiel 1 hergestellten Präparat.

Ausbeute : 1,04 g (30 % der Theorie).

Beispiel 8

5,11-Dihydro-11-[(4-methyl-1-piperazinyl)-acetyl]-6H-dibenz-[b,e]azepin-6-on

Hergestellt analog Beispiel 1 durch Umsetzung von 10 g (0,048 Mol) 5,11-Dihydro-6H-dibenz[b,e]azepin-6-on, 50 g (0,12 Mol) Phenyllithium (20 %ig in Benzol/Äther) und 20,5 g (0,096 Mol) 4-Methyl-1-piperazin-essigsäure-butylester in 200 ml absolutem Tetrahydrofuran in einer Ausbeute von 4,2 g (25 % der Theorie).

Die Verbindung ist nach Mischschmelzpunkt, Dünnschichtchromatogramm und IR-Spektrum identisch mit dem nach Beispiel 2 hergestellten Präparat.

Beispiel 9

5,11-Dihydro-11-[(1-methyl-4-piperidinyl)acetyl]-6H-dibenz-,[b,e]azepin-6-on

Hergestellt analog Beispiel 1 durch Umsetzung von 5,0 g (0,024 Mol) 5,11-Dihydro-6H-dibenz[b,e]azepin-6-on, 38 ml (0,06 Mol) n-Butyllithium (1,6 molar in Hexan) mit 11,9 g (0,048 Mol) 1-Methyl-4-piperidinessigsäure-benzylester in 100 ml absolutem Tetrahydrofuran in einer Ausbeute von 1,7 g (20 % der Theorie).

Die Verbindung ist nach Mischschmelzpunkt, Dünnschichtchromatogramm und IR-Spektrum identisch mit dem nach Beispiel 1 hergestellten Präparat.

Im folgenden wird die Herstellung pharmazeutischer Anwendungsformen anhand einiger Beispiele beschrieben :

Beispiel I

Tabletten mit 25 mg 5,11-Dihydro-11-[[(1-methyl-4-piperidinyl)amino]carbonyl]-6H-dibenz[b,e]azepin-6-on

Zusammensetzung
1 Tablette enthält

| | |
|---|---|
| Wirkstoff | 25,0 mg |
| Milchzucker | 148,0 mg |
| Kartoffelstärke | 65,0 mg |
| Magnesiumstearat | 2,0 mg |
| | 240,0 mg |

Herstellungsverfahren

Aus Kartoffelstärke wird durch Erwärmen ein 10 %iger Schleim hergestellt. Die Wirksubstanz, Milchzucker und die restliche Kartoffelstärke werden gemischt und mit obigem Schleim durch ein Sieb der Maschenweite 1,5 mm granuliert. Das Granulat wird bei 45 °C getrocknet, nochmals durch obiges Sieb gerieben, mit Magnesiumstearat vermischt und zu Tabletten verpreßt.

Tablettengewicht : 240 mg

Stempel : 9 mm

## Beispiel II

Dragées mit 25 mg 5,11-Dihydro-11-[[(1-methyl-4-piperidinyl)amino]carbonyl]-6H-dibenzo[b,e]azepin-6-on

Die nach Beispiel I hergestellten Tabletten werden nach bekanntem Verfahren mit einer Hülle überzogen, die im wesentlichen aus Zucker und Talkum besteht. Die fertigen Dragées werden mit Hilfe von Bienenwachs poliert.

Dragéegewicht : 300 mg

## Beispiel III

Ampullen mit 1 mg 5,11-Dihydro-11-[[(1-methyl-4-piperidinyl)amino]carbonyl]-6H-dibenz[b,e]azepin-6-on-hydrochlorid

Zusammensetzung
1 Ampulle enthält :

| | |
|---|---|
| Wirkstoff | 1,0 mg |
| Natriumchlorid | 8,0 mg |
| Dest. Wasser ad | 1 ml |

Herstellungsverfahren

Die Wirksubstanz und Natriumchlorid werden in dest. Wasser gelöst und anschließend auf das gegebene Volumen aufgefüllt. Die Lösung wird sterilfiltriert und in 1 ml-Ampullen abgefüllt.

Sterilisation : 20 Minuten bei 120 °C.

## Beispiel IV

Suppositorien mit 25 mg 5,11-Dihydro-11-[[(1-methyl-4-piperidinyl)amino]carbonyl]-6H-dibenz[b,e]azepin-6-on

Zusammensetzung
1 Zäpfchen enthält :

| | |
|---|---|
| Wirkstoff | 5,0 mg |
| Zäpfchenmasse (z. B. Witepsol W 45[R] | 1 695,0 mg |
| | 1 700,0 mg |

Herstellungsverfahren

Die feinpulverisierte Wirksubstanz wird in der geschmolzenen und auf 40 °C abgekühlten Zäpfchenmasse suspendiert. Man gießt die Masse bei 37 °C in leicht vorgekühlte Zäpfchenformen aus.

Zäpfchengewicht : 1,7 g

## Beispiel V

Tropfen mit 0,5 g 5,11-Dihydro-11-[[(1-methyl-4-piperidinyl)amino]carbonyl]-6H-dibenzo[b,e]azepin-6-on-hydrochlorid pro 100 ml Lösung

Zusammensetzung
100 ml Tropflösung enthalten :

| | |
|---|---|
| p-Hydroxybenzoesäuremethylester | 0,035 g |
| p-Hydroxybenzoesäurepropylester | 0,015 g |
| Anisöl | 0,05 g |
| Menthol | 0,06 g |

| | |
|---|---|
| Ethanol rein | 10,0 g |
| Wirkstoff | 0,5 g |
| Natriumcyclamat | 1,0 g |
| Glycerin | 15,0 g |
| Dest. Wasser | ad 100,0 ml |

Herstellungsverfahren

Die Wirksubstanz und Natriumcyclamat werden in ca. 70 ml Wasser gelöst und Glycerin zugefügt. Man löst die p-Hydroxybenzoesäureester, Anisöl sowie Menthol in Ethanol und fügt diese Lösung unter Rühren der wäßrigen Lösung zu. Abschließend wird mit Wasser auf 100 ml aufgefüllt und schwebeteilchenfrei filtriert.

**Patentansprüche**

1. Substituierte 5,11-Dihydro-6H-dibenz[b,e]azepin-6-one der allgemeinen Formel I

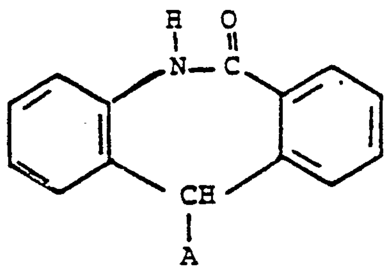

(I)

in der A eine (1-Methyl-4-piperidinyl)acetyl-, (4-Methyl-1-piperazinyl)acetyl- oder [(1-Methyl-4-piperidinyl)amino]carbonylgruppe bedeutet, ihre enantiomeren Formen und ihre physiologisch verträglichen Säureadditionssalze mit anorganischen oder organischen Säuren.

2. 5,11-Dihydro-11-[[(1-methyl-4-piperidinyl)amino]carbonyl]-6H-dibenz[b,e]azepin-6-on und ihre pharmakologisch verträglichen Säureadditionssalze mit anorganischen oder organischen Säuren.

3. 5,11-Dihydro-11-[(1-methyl-4-piperidinyl)acetyl]-6H-dibenz[b,e]azepin-6-on und ihre pharmakologisch verträglichen Säureadditionssalze mit anorganischen oder organischen Säuren.

4. 5,11-Dihydro-11-[(4-methyl-1-piperazinyl)acetyl]-6H-dibenz[b,e]azepin-6-on und ihre pharmakologisch verträglichen Säureadditionssalze mit anorganischen oder organischen Säuren.

5. Arzneimittel, enthaltend eine oder mehrere Verbindungen der allgemeinen Formel I gemäß Anspruch 1 neben den üblichen Träger- und/oder Hilfsstoffen.

6. Verfahren zur Herstellung neuer, substituierter 5,11-Dihydro-6H-dibenz[b,e]azepin-6-one der allgemeinen Formel I

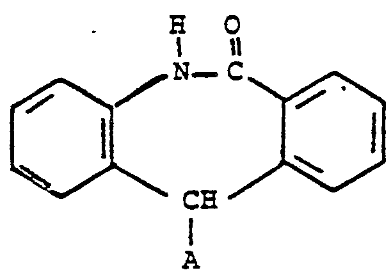

(I)

in der A die (1-Methyl-4-piperidinyl)acetyl- oder (4-Methyl-1-piperazinyl)acetylgruppe bedeutet, und von deren Salzen mit anorganischen oder organischen Säuren, dadurch gekennzeichnet, daß das 5,11-Dihydro-6H-dibenz[b,e]azepin-6-on der Formel II

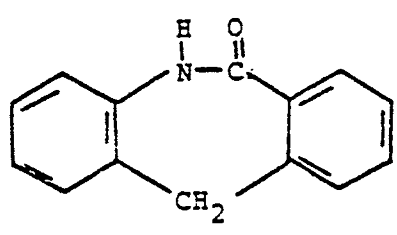

(II)

in sein Di-Lithiumsalz übergeführt und dieses anschließend mit einem Ester der allgemeinen Formel III

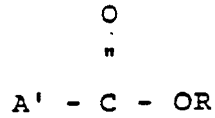

(III)

in der

A die (1-Methyl-4-piperidinyl)methyl- oder (4-Methyl-1-piperazinyl)-methylgruppe bedeutet und

R einen Alkylrest mit 1 bis 10 Kohlenstoffatomen oder einen Aralkylrest mit 7 bis 13 Kohlenstoffatomen darstellt, umgesetzt und eine so erhaltene Verbindung der allgemeinen Formel I gewünschtenfalls anschließend in ihre Säureadditionssalze mit anorganischen oder organischen Säuren übergeführt wird.

7. Verfahren zur Herstellung von 5,11-Dihydro-11-[[(1-methyl-4-piperidinyl)amino]carbonyl]-6H-dibenz[b,e]-azepin-6-on und von dessen Salzen mit anorganischen oder organischen Säuren, dadurch gekennzeichnet, daß ein reaktives Derivat der Carbonsäure der Formel IV

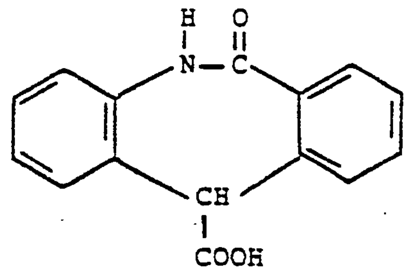

(IV)

mit 1-Methyl-4-amino-piperidin umgesetzt und das erhaltene Produkt gegebenenfalls in seine Säureadditionssalze mit anorganischen oder organischen Säuren übergeführt wird.

8. Verfahren gemäß Anspruch 6, dadurch gekennzeichnet, daß die Verbindung der Formel II mit N-Butyllithium, gegebenenfalls in Gegenwart von Tetramethylethylendiamin, mit tertiär-Butyllithium, Lithiumdiisopropylamid, Lithiumdicyclohexylamid oder Lithiumphenyl in einem organischen inerten Lösungsmittel bei Temperaturen zwischen —60 °C und 0 °C in ihr Di-Lithiumsalz übergeführt wird und die anschließende Umsetzung mit einem Ester der allgemeinen Formel III in demselben Reaktionsgemisch bei Temperaturen bis zur Raumtemperatur erfolgt.

9. Verfahren gemäß Anspruch 7, dadurch gekennzeichnet, daß als reaktive Derivate der Carbonsäure der Formel IV ihre Säurehalogenide, gemischten Anhydride, ihre Umsetzungsprodukte mit N,N′-Carbonyldiimidazol oder mit N,N′-Dicyclohexylcarbodiimid, oder ihre Ester mit aliphatischen Alkoholen von 1 bis 8 Kohlenstoffatomen oder mit araliphatischen Alkoholen mit 7 bis 13 Kohlenstoffatomen mit 1-Methyl-4-amino-piperidin in Gegenwart inerter organischer Lösungsmittel oder in Gegenwart eines Überschusses dieses Piperidins zwischen Zimmertemperatur und dem Siedepunkt des Reaktionsgemisches umgesetzt wird.

10. Verwendung einer Verbindung der allgemeinen Formel I gemäß Anspruch 1 zur Herstellung eines Arzneimittels zur Bekämpfung von Erkrankungen des Magen-Darm-Traktes.

## Claims

1. Substituted 5,11-dihydro-6H-dibenz[b,e]azepin-6-ones of general formula I

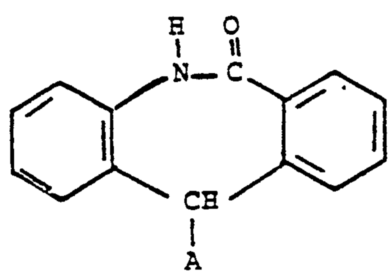

(I)

wherein A represents a (1-methyl-4-piperidinyl)acetyl, (4-methyl-1-piperazinyl)acetyl or [(1-methyl-4-piperidinyl)amino]carbonyl group, the enantiomeric forms thereof and the physiologically acceptable addition salts thereof with inorganic or organic acids.

2. 5-11-Dihydro-11-[[(1-methyl-4-piperidinyl)amino]-carbonyl]-6H-dibenz[b,e]azepin-6-one and the pharmacologically acceptable acid addition salts thereof with inorganic or organic acids.

3. 5,11-Dihydro-11-[(1-methyl-4-piperidinyl)acetyl]-6H-dibenz[b,e]azepin-6-one and the pharmacologically acceptable acid addition salts thereof with inorganic or organic acids.

4. 5,11-Dihydro-11-[(4-methyl-1-piperazinyl)acetyl]-6H-dibenz[b,e]azepin-6-one and the pharmacologically acceptable acid addition salts thereof with inorganic or organic acids.

5. Pharmaceutical compositions containing one or more compounds of general formula I as claimed in claim 1 together with conventional carriers and/or excipients.

6. Process for preparing new substituted 5,11-dihydro-6H-dibenz[b,e]azepin-6-ones of general formula I

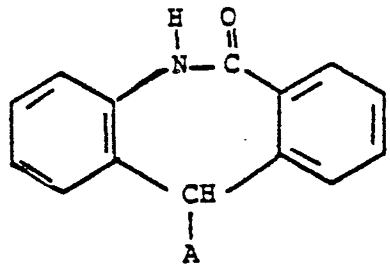

(I)

wherein A represents a (1-methyl-4-piperidinyl)acetyl or (4-methyl-1-piperazinyl)acetyl group, and the salts thereof with inorganic or organic acids, characterised in that the 5,11-dihydro-6H-dibenz[b,e]azepin-6-one of formula II

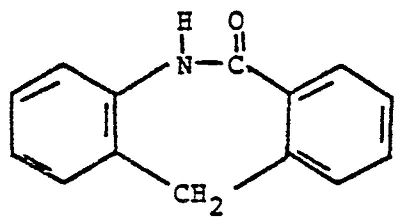

(II)

is converted into the dilithium salt thereof and this is subsequently reacted with an ester of general formula III

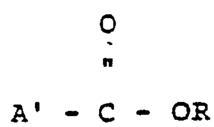

(III)

wherein

A represents a (1-methyl-4-piperidinyl)methyl or (4-methyl-1-piperazinyl)-methyl group and

R represents an alkyl group with 1 to 10 carbon atoms or an aralkyl group with 7 to 13 carbon atoms, and a compound of general formula I thus obtained is subsequently, if desired, converted into the acid addition salts thereof with inorganic or organic acids.

7. Process for preparing 5,11-dihydro-11-[[(1-methyl-4-piperidinyl)amino]carbonyl]-6H-dibenz[b,e]-azepin-6-one and the salts thereof with inorganic or organic acids, characterised in that a reactive derivative of the carboxylic acid of formula IV

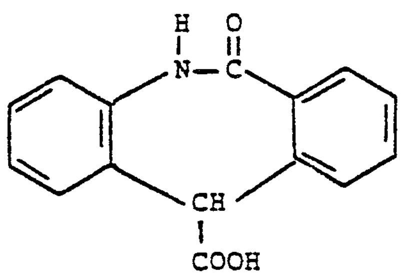

(IV)

is reacted with 1-methyl-4-amino-piperidine and the resulting product is optionally converted into the acid addition salts thereof with inorganic or organic acids.

8. Process as claimed in claim 6, characterised in that the compound of formula II is converted into the dilithium salt thereof with N-butyllithium, optionally in the presence of tetramethyl ethylenediamine, with tertiary butyllithium, lithium diisopropylamide, lithium dicyclohexylamide or lithium phenyl in an organic inert solvent at temperatures of between — 60 °C and 0 °C and the subsequent reaction with an

ester of general formula III is carried out in the same reaction mixture at temperatures up to ambient temperature.

9. Process as claimed in claim 7, characterised in that the carboxylic acid of formula IV, its acid halide, mixed anhydride, reaction product with N,N'-carbonyldiimidazole or with N,N'-dicyclohexylcarbodiimide, or ester thereof with aliphatic alcohols having 1 to 8 carbon atoms or with araliphatic alcohols with 7 to 13 carbon atoms, is reacted with 1-methyl-4-amino-piperidine in the presence of inert organic solvents or in the presence of an excess of this piperidine at between ambient temperature and the boiling point of the reaction mixture.

10. Use of a compound of general formula I as claimed in claim 1 for preparing a pharmaceutical composition for treating diseases of the gastrointestinal tract.

**Revendications**

1. 5,11-dihydro-6H-dibenz[b,e]azépin-6-ones substituées répondant à la formule générale

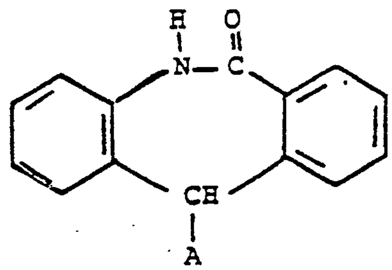

(I)

dans laquelle A désigne un groupe (1-méthyl-4-pipéridinyl)acétyle, (4-méthyl-1-pipérazinyl)acétyle ou [(1-méthyl-4-pipéridinyl)amino]-carbonyle, leurs formes énantiomères et leurs sels d'addition aux acides minéraux ou organiques physiologiquement acceptables.

2. 5,11-dihydro-11-[[(1-méthyl-4-pipéridinyl)-amino]carbonyl]-6H-dibenz[b,e]azépin-6-one et ses sels d'addition aux acides minéraux ou organiques pharmacologiquement acceptables.

3. 5,11-dihydro-11-[(1-méthyl-4-pipéridinyl)acéthyl]-6H-dibenz[b,e]azépin-6-one et ses sels d'addition aux acides minéraux ou organiques pharmacologiquement acceptables.

4. 5,11-dihydro-11-[(4-méthyl-1-pipérazinyl)acéthyl]-6H-dibenz[b,e]azépin-6-one et ses sels d'addition aux acides minéraux ou organiques pharmacologiquement acceptables.

5. Médicaments contenant un ou plusieurs composés répondant à la formule générale I suivant la revendication 1 en plus des supports et/ou adjuvants habituels.

6. Procédé de préparation de nouvelles 5,11-dihydro-6H-dibenz[b,e]azépin-6-ones substituées répondant à la formule I

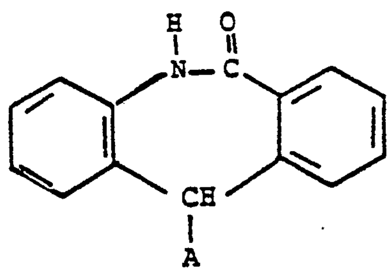

(I)

dans laquelle A désigne le groupe (1-méthyl-4-pipéridinyl)acétyle ou le groupe (4-méthyl-1-pipérazinyl)acétyle et de leurs sels avec des acides minéraux ou organiques, caractérisé en ce qu'on transforme la 5,11-dihydro-6H-dibenz[b,e]azépin-6-one répondant à la formule II

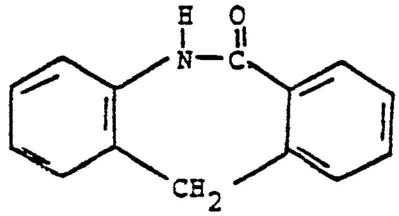

(II)

en son sel de dilithium, et en ce qu'on fait réagir ensuite celui-ci avec un ester répondant à la formule générale III

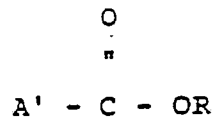

(III)

dans laquelle

A désigne le groupe (1-méthyl-4-pipéridinyl)méthyle ou (4-méthyl-1-pipérazinyl)méthyle, et

R désigne un radical alkyle en $C_1$ à $C_{10}$ ou un radical aralkyle en $C_7$ à $C_{13}$, et en ce qu'on transforme si on le désire ensuite un composé répondant à la formule générale I ainsi obtenu en ses sels d'addition aux acides avec des acides minéraux ou organiques.

7. Procédé de préparation de la 5,11-dihydro-11-[[(1-méthyl-4-pipéridinyl)amino]carbonyl]-6H-dibenz[b,e]azépin-6-one et de ses sels avec des acides minéraux ou organiques, caractérisé en ce qu'on fait réagir un dérivé réactif de l'acide carboxylique répondant à la formule IV

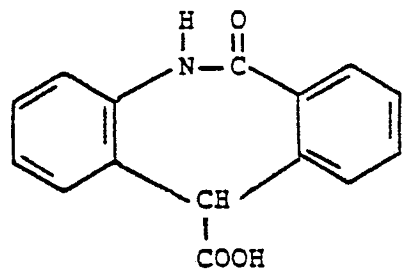

(IV)

avec la 1-méthyl-4-amino-pipéridine, et en ce qu'on transforme le cas échéant le produit obtenu en ses sels d'addition aux acides avec des acides minéraux ou organiques.

8. Procédé suivant la revendication 6, caractérisé en ce qu'on transforme le composé répondant à la formule II avec du N-butyl-lithium, le cas échéant en présence de tétraméthyléthylènediamine, avec du tert-butyl-lithium, du diisopropylamidure de lithium, du cyclohexylamidure de lithium ou du lithium-phényle dans un solvant organique inerte à des températures comprises entre — 60 °C et 0 °C en son sel de dilithium, et en ce que la réaction ultérieure avec un ester répondant à la formule générale III s'effectue dans le même mélange réactionnel à des températures allant jusqu'à la température ambiante.

9. Procédé suivant la revendication 7, caractérisé en ce qu'on fait réagir, comme dérivés réactifs de l'acide carboxylique répondant à la formule IV, ses halogénures d'acide, ses anhydrides mixtes, ses produits de réaction avec le N,N'-carbonyldiimidazole ou avec le N,N'-dicyclohexylcarbodiimide, ou ses esters avec des alcools aliphatiques en $C_1$ à $C_8$ ou avec des alcools araliphatiques en $C_7$ à $C_{13}$ avec la 1-méthyl-4-amino-pipéridine en présence de solvants organiques inertes ou en présence d'un excès de cette pipéridine entre la température ambiante et le point de fusion du mélange réactionnel.

10. Utilisation d'un composé répondant à la formule générale I suivant la revendication 1 pour la préparation d'un médicament pour lutter contre les maladies du tractus gastro-intestinal.